# EUROPEAN PATENT SPECIFICATION

(11) **EP 0 496 756 B1**
(45) Date of publication and mention of the grant of the patent: **23.07.1997**
(21) Application number: 90915068.2
(22) Date of filing: 16.10.1990
(51) Int. Cl.: G08B 21/00

(54) **METHOD AND APPARATUS FOR THE TREATMENT AND PREVENTION OF POSTURE DEFICIENCIES OF THE SPINE**
VERFAHREN UND VORRICHTUNG FÜR DIE BEHANDLUNG UND VERHINDERUNG VON RÜCKGRATHALTUNGSMÄNGELN
PROCEDE ET APPAREIL DE TRAITEMENT ET DE PREVENTION DES TROUBLES POSTURAUX DE LA COLONNE VERTEBRALE

(30) Priority: 16.10.1989 AU 6865/89
(43) Date of publication of application: 05.08.1992
(73) Proprietor: LARBACK PTY. LTD., Southport, Queensland 4215 (AU)
(72) Inventor: CARTER, Michael, Anthony, Mansfield, QLD 4122 (AU)
(74) Representative: Patentanwälte Wenzel & Kalkoff
(86) International application number: AU9000495
(87) International publication number: WO9106082

(56) References cited:
- AU-B- 3 373 484
- FR-A- 2 591 472
- US-A- 3 582 935
- US-A- 3 608 541
- US-A- 3 644 919
- US-A- 3 670 320
- US-A- 4 191 949
- US-A- 4 392 126
- US-A- 4 801 921
- US-A- 4 846 462

## Description

This invention relates to a method and apparatus for the treatment and prevention of posture deficiencies of the spine. This invention has particular application to such methods and apparatus as may be suitable for the encouragement of adoption of a desired posture amongst patients with no underlying physical impairment, and the invention will be described with reference to this application. However, this invention may find use in other applications such as the treatment of physically impaired persons for posture insufficiency to achieve a more normal posture.

The prevention and treatment of back problems including those caused by poor posture and poor work practices is an ongoing medical problem of our society, the considerable cost of which contributes indirectly to the current cost of goods and services generally. It is recognized that the losses attributable to back problems cost business and industry a considerable amount of money, and that the costs associated with medical-grounds retirements, retraining and compensation as a result of workplace induced back injury are increasing. Methods of treatment of posture induced back problems amongst patients have included physiotherapy and the use of prostheses such as back braces, corsets or the like. These have proved to be deficient in a large number of cases since they tend to be palliative rather than curative and do not address the fundamental cause of the injuries in the first place.

US-A-3608541 discloses apparatus comprising a plurality of segments retained in contact with the spine of the wearer and having at their outer extremity a conduit adapted to pass a cable. The cable is anchored to the uppermost segment and to an indicator, whereby movement of the spine to a position of poor posture tends to alter the length of the cable, thus effecting triggering of the indicator. A disadvantage of this approach is that the apparatus will not detect subtle changes in lumbar lordosis, and will not detect and indicate complementary changes in regions of the spine which maintain approximately the same effective length of the cable.

Prevention of back injury through poor posture in the workplace has also been directed somewhat away from the major cause of the injury, although the increasing provision of ergonomic work stations and revision of longstanding poor work practices has ameliorated some problems in some areas.

Poor posture may be regarded as being of two broad types, the first being an acquired habit of poor posture and the second being attributable to the physical characteristics of the patient. The prior art methods and apparatus for treatment appear to be deficient in the prevention and/or cure of both types. For example, it has been found that the existing methods of treatment, such as back braces and corsets, are ineffective because they do not treat or rectify the frequent cause of the spinal posture deficiency, that is, a muscle imbalance of the opposing muscle groups which hold the spine in its correct posture. Braces and corsets do nothing to rectify this muscle imbalance because they merely hold the spine in its correct posture. This does not condition the muscles to support the spine but rather allows the muscles to deteriorate further because they are kept inactive.

Braces and corsets also have other disadvantages such as the wearer's inconvenience of movement restriction, the interference with clothing, and their poor appearance. The wearer, unable to bend at the waist, cannot perform many activities necessary throughout the day. Simple functions such as tying shoelaces or picking up dropped items become arduous tasks. Additionally, the bending of the back may in fact be advantageous in promoting nutrition of the inter-vertebral discs.

A further problem resides in the fact that the inter-vertebral discs have a negligible nerve supply. Therefore, under normal circumstances, they feel no pain. When a person habitually assumes incorrect posture (whether sitting, standing or lifting) damage is being done unwittingly because there is no pain to act as a warning of impending damage. Compounding this problem is that the damage is cumulative.

The present invention aims to substantially alleviate the above disadvantages and to provide methods and apparatus which will be reliable and efficient in use. Other objects and advantages of this invention will hereinafter become apparent.

Accordingly, in one aspect the present invention resides broadly in a method for the prevention and treatment of posture deficiencies of the spine, of the type including continuously monitoring the posture of a person, sensing the onset of poor posture, and signalling the sensed poor posture, characterised by
continuously monitoring a plurality of points on a portion of the spine by
sensing with a plurality of contacts the relation in position between adjacent said monitored points, and
signalling the user where said sensed relation indicates a poor posture condition of said portion.

The monitoring of the person's posture may be by any suitable means consistent with the desired function.
Preferably, the monitoring is accomplished by monitoring means mounted unobtruively to the person at or about the location of the bodily part or parts which tend towards creating the poor posture to be prevented. For example, the monitoring means may comprise an article configurable to the shape of the relevant body part and adapted to be attached to the person under clothing, and mounting the sensing means.

The means of sensing the onset of poor posture may take any suitable form including electromechanical switch sensing, pressure transducer sensing or the like. The signalling function may also be provided by any suitable means, including vibratory stimuli, audio stimuli, electical stimuli or a visual indication such as indication lights. It is preferred, however, that the signalling be of an unpleasant or irritating nature to reinforce the maintenance of good posture and to this end it is preferred to utilize a method which involves vibro-tactile stimulus of sub audible frequency.

In a further aspect, this invention resides broadly in therapeutic apparatus including:
a body member,
sensing means associated with said body member, wherein
said body member is adapted to be secured to a first point on a selected portion of the spine of the wearer, said sensing means is adapted to monitor with a plurality of contacts at least one other point on said selected portion, and said apparatus further comprises reacting means responsive to the relative positions of said body member and said respective sensing means and adapted to react to a selected said relative position.

The body member may take any form consistent with the function of maintaining the sensing means in a position to monitor the required condition, and as such the form of the body member may be dictated by the condition, the part of the body and the nature of the sensing means. For example, where the condition to be monitored relates to the configuration of the wearer's spine, the body member may take the form of a strip or the like adapted to be disposed along the portion of the spine to be monitored. The strip may be configurable or adjustable such that it may be useful on several bodily locations or be useful for different wearers. For example, the strip may be of a material or construction such that it will retain a selected shape in one application, but may be deformed or reconfigured to be useful in other applications.

The securing means may also take any form consistent with the function of maintaining the body member in position on the wearer. For example, the securing means may comprise adhesive pads or tape members, straps or any other suitable means. Preferably the securing means, and indeed the body member and securing means in assembly, are such that they are unobtrusive to avoid embarrassment of the wearer in use. In certain embodiments of the present invention, the securing means may adjustably mount the body member to provide an element of fine tuning to the positioning the body member relative to the part to be monitored.

The sensing means may take any suitable form dependent upon the nature of the condition to be monitored. For example, the sensing means, in embodiments adapted to monitor curvature of the spine, may include means to continuously provide a signal or other output reflecting the curvature. In other embodiments, the sensing means may only provide a signal in response to the, for example, curvature of the spine exceeds certain predetermined limits. Accordingly, the sensing means may be selected from a wide array of sensing means including pressure, temperature, conductivity or proximity sensing means.

The sensing means comprises a set of electrical contacts mounted within or to the body member at a point whereby operation of the contacts occurs when an adverse posture condition prevails. Preferably, in the case of apparatus responsive to spinal curvature, the body member may be provided with more than one set of contacts to detect opposing defects or to ensure that the required signal is generated from at least one of the sensing means. The sensing means may be adapted for use in a powered circuit such that the opening or closing of the preferred contacts provides a current or the interruption of current, the change of state being useful to drive or trigger the reacting means. Alternatively, the contacts may involve negligible current flow such as may be the case where the potential difference between the contacts is being measured. Of course, any other suitable sensing means may be used, including magnetic sensing means such as reed switches or inductance devices and accordingly the sensing means may include a body mounted portion acting in conjunction with, for example, a magnetic component attached to the wearer.

The reacting means may take any form consistent with its being operable in response to the selected sensing means. For example, the reacting means may comprise means of recording condition status on a continuous, intermittent or condition responsive basis. Alternatively, the reacting means may serve to signal the wearer of the condition such that positive reinforcement of, for example, poor posture habits may be attained. Of course, the apparatus may be so configured that both condition responsive and timed stimuli are supplied to the wearer, to indicate poor posture and to stimulate the wearer periodically for voluntary maintenance of good posture respectively. The timed stimuli of such apparatus may be user programmable to act as an unobtrusive alarm or reminder system.

Where the reacting means is adapted to provide a stimulus to the wearer, it is preferred that the stimulus be detectable only by the wearer such that the apparatus is not obtrusive in use. The stimulus may take the form of a mild electric shock, thermal indication, visual indication or soft, audible indication, or any other suitable indication. However, it is preferred that the reacting means include means capable of generating a vibration to which the wearer's tactile sense can respond, the vibration preferably being of sub-audible frequency and/or volume such that the vibration cannot be detected by others.

The reacting means may be located on the wearer's body, either together with the body member or the securing means or independently thereof. The apparatus may also include a plurality of reacting means, which may be configured to operate simultaneously or, if desired, may be configured to provide alternate indications to the wearer according to the direction indicated by the sensing means.

A power supply is preferably provided to power the circuitry associated with the preferred apparatus. The power supply may be integral with the body member or securing means, or may be located apart therefrom and connected by suitable wires or the like, such that the power supply may be carried in a pocket.

When used in a rehabilitatory role or posture training role, the apparatus may advantageously be provided with a counter or the like adapted to record the number of stimuli applied to the wearer by the reacting means.

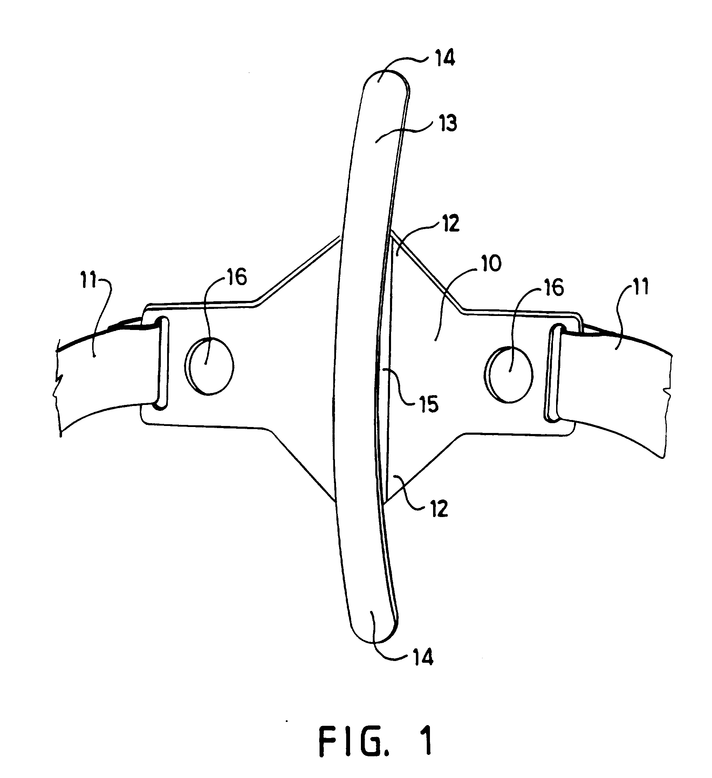

The invention will be further described with reference to a preferred embodiment thereof as illustrated in the accompanying drawings, wherein:-
FIG. 1 is a perspective view of apparatus in accordance with the present invention, and

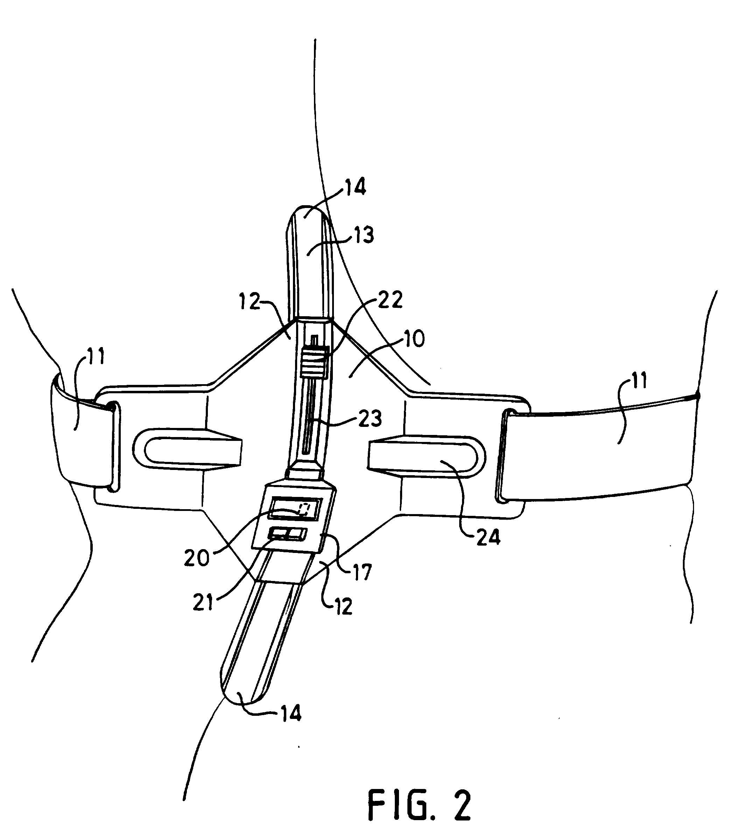

FIG. 2 is a reverse perspective view of the apparatus of FIG. 1 in use.

In the figures there is provided apparatus suitable for use in the lumbar region of the wearers back and including a body member 10 of plastics material and adapted to be supported in the region of a wearers back by securing straps 11 which may be secured by any suitable means (not shown).

The body member 10 is provided with extended regions 12 adapted to increase the dimension of the body member 10 along the spine.

Pivotally mounted to the extended regions 12 of the body member 10 is a flexible strip 13 which is curved such that it conforms with and lies against the spine of the wearer in use. The flexibility of the strip 13 is such that the strip may conform to changes in the curvature of the spine of the wearer. The curvature of the flexible strip 13 and its hinged arrangement to the extended regions 12 of the body member 10, provide for a clearance space 15 within which is disposed a micro-switch (not shown).

Mounted to the body member 10 and adjacent to users skin in use are a pair of vibro-tactile emitters 16 which are controlled by the micro-switch and powered by a power supply 17 mounted on the body member 10. The power supply has formed integrally therewith a liquid crystal display 20 and control buttons 21 to register the number of times the vibro-tactile emitters 16 are called into play.

The contact point of the micro-switch with the flexible strip 13 may be varied and the apparatus hence adjusted by mounting of the micro-switch on sliding control knob 22 adapted to move the micro-switch to points of varying clearance in the clearance space 15. The knob is constrained in its movement by slot 23.

In order to protect the rear of the vibro-tactile emitters 16, there are provided integrally moulded hollow webs 24.

In use, unsatisfactory spinal posture is detected by a flexible strip 13 being deformed by the spine, which results in closing of the micro-switch, which in turn causes the power supply to drive the vibro-tactile emitters 16. The sensitivity of the apparatus is selected by moving the sliding control knob 22 within the slot 23. When the lumbar curve flattens to a predetermined point for the stimulus to be emitted, the somewhat annoying vibration of the vibro-tactile emitters is transmitted to the skin of the user.

The counter display records the number of times the vibratory stimulus is emitted for the purpose of progress monitoring such that the conditioned response of the wearer should decrease the frequency that the vibratory stimulus is emitted. As the muscle groups become conditioned to holding the spine in its optimum position.

Apparatus in accordance with the above described embodiment utilizes a conditioned response feedback method to condition the muscles which stabilizes the spine thereby preventing or treating poor posture. Since no physical support is provided, the apparatus makes the muscles do the work thereby strengthening the muscles. The silent vibratory stimulus emitted by the apparatus onto the surface of the skin of the wearer is mildly unpleasant, thus reinforcing the conditioned response. Accordingly, apparatus in accordance with the above embodiment does not simply encourage a straight back, but rather a fit and healthy back.

This particular embodiment was also provided with means to issue a vibration at selectable pre-determined intervals, for example, every thirty (30) minutes in a distinct stimulus pattern as a reminder indicating that the wearer should bend and stretch gently and briefly to aid nutrition of the intervertebral discs.

Apparatus of the abovedescribed kind is neither physically nor socially inhibiting since nothing is able to be seen or heard by others. It does not interfere with clothing and it allows the normal range of movement, for example, the wearer can bend over if necessary to tie shoelaces, pick up objects and the like.

Whilst apparatus is described with reference to the problems occurring in the lumbar region of the spine, the apparatus is in principle, equally applicable to the cervical and upper thoracic regions of the spine as well as in lateral curvature conditions such as scoliosis.

Whilst the above has been given by way of description of a preferred embodiment of the present invention, this should not be taken to limit the invention as defined by the claims appended hereto.

## Claims

1. A method for the prevention and treatment of posture deficiencies of the spine, of the type including continuously monitoring the posture of a person, sensing the onset of poor posture, and signalling the sensed poor posture, characterized by
continuously monitoring a plurality of points on a portion of the spine by
sensing with a plurality of contacts the relation in position between adjacent said monitored points, and
signalling the user where said sensed relation indicates a poor posture condition of said portion.

2. A method according to Claim 1, wherein said signalling is provided by means of a vibro-tactile stimulus of sub audible frequency.

3. Therapeutic apparatus including:
a body member (10),
sensing means (14) associated with said body member (10),
characterized in that
said body member (10) is adapted to be secured to a first point on a selected portion of the spine of the wearer, said sensing means (14) is adapted to monitor with a plurality of contacts at least one other point on said selected portion, and
said apparatus further comprises reacting means (16) responsive to the relative positions of said body member (10) and said respective sensing means (14) and adapted to react to a selected said relative position.

4. Therapeutic apparatus according to Claim 3, wherein said body member includes a strip or the like adapted to be disposed along the portion of the spine of a wearer.

5. Therapeutic apparatus according to any one of Claims 3 or 4, wherein said securing means is selected from adhesive pads, adhesive tape members, or straps.

6. Therapeutic apparatus according to Claim 5, wherein said sensing means includes means to continuously provide a signal or other output reflecting the curvature of the spine of the wearer.

7. Therapeutic apparatus according to Claim 5, wherein said sensing means provides a signal in response to the curvature of the spine of the wearer exceeding predetermined limits.

8. Therapeutic apparatus according to Claim 7, wherein said sensing means comprises a set of electrical contacts mounted within or to the body member such that said contacts are operable when an adverse posture condition prevails.

9. Therapeutic apparatus according to any one of Claims 3 to 8, wherein said reacting means serves provide a stimulus to the wearer.

10. Therapeutic apparatus according to Claim 9, wherein said stimulus is detectable only by the wearer.

11. Therapeutic apparatus according to Claim 10, wherein said stimulus is provided by reacting means capable of generating a vibration to which the wearer's tactile sense can respond.

12. Therapeutic apparatus according to Claim 11, wherein said vibration is of a sub-audible frequency and/or volume such that the vibration cannot be detected by others.

13. Therapeutic apparatus according to any one of Claims 9 to 12, wherein there is further provided a counter or the like adapted to record the number of stimulations applied to the wearer by said reacting means.

## Patentansprüche

1. Ein Verfahren zum Verhindern und Behandeln von Haltungsschwächen des Rückgrats, und zwar der folgende Schritte umfassenden Art: Ständiges Überwachen der Haltung eines Menschen, Wahrnehmen des Beginns schlechter Haltung und Signalisieren wahrgenommener schlechter Haltung, gekennzeichnet durch:
Kontinuierliches Überwachen einer Mehrzahl Stellen oder Punkte an einem Abschnitt des Rückgrats durch Wahrnehmen der relativen Lage zwischen benachbarten der überwachten Stellen mittels einer Mehrzahl Berührungspunkte und
Signalisieren an den Benutzer, wenn die wahrgenommene Relation einen schlechten Haltungszustand dieses Abschnitts erkennen läßt.

2. Ein Verfahren nach Anspruch 1, bei dem das Signalisieren mittels einer vibrotaktilen Stimulierung mit Unterhörfrequenz veranlaßt wird.

3. Therapeutisches Gerät umfassend:
Ein Teil (10) für den Körper,
dem Teil (10) für den Körper zugeordnete Wahrnehmungsmittel (14),
dadurch gekennzeichnet, daß das Teil (10) für den
Körper ausgebildet ist, an einer ersten Stelle auf einem ausgewählten Abschnitt des Rückgrats des Trägers befestigt zu werden,
das Wahrnehmungsmittel (14) ausgebildet ist, um mittels einer Mehrzahl Berührungspunkte mindestens eine andere Stelle an dem ausgewähltem Abschnitt zu überwachen, und
das Gerät weiter ein Reaktionsmittel (16) umfapt, das auf die relativen Lagen des Teils (10) für den Körper und des entsprechenden Wahrnehmungsmittels (14) anspricht und ausgebildet ist, auf eine ausgewählte relative Lage zu reagieren.

4. Therapeutisches Gerät nach Anspruch 3, bei dem das Teil für den Körper einen Streifen oder dergleichen umfaßt, der entlang des Abschnitts des Rückgrats eines Trägers angeordnet werden kann.

5. Therapeutisches Gerät nach einem der Ansprüche 3 oder 4, bei dem das Befestigungsmittel aus Klebepolstern, Klebebandteilen oder Bändern ausgewählt ist.

6. Therapeutisches Gerät nach Anspruch 5, bei dem das Abtastmittel Mittel umfaßt, um kontinuierlich ein Signal oder einen anderen Ausgang bereitzustellen, die die Rückgratkrümmung des Trägers wiedergeben.

7. Therapeutisches Gerät nach Anspruch 5, bei dem das Wahrnehmungsmittel ein Signal als Ergebnis auf die vorbestimmte Grenzen überschreitende Rückgratkrümmung des Trägers bereitstellt.

8. Therapeutisches Gerät nach Anspruch 7, bei dem das Wahrnehmungsmittel einen Satz elektrischer Kontakte umfaßt, die innerhalb des Teils für den Körper oder an demselben so angebracht sind, daß die Kontakte betriebsfähig sind, wenn ein schlechter Haltungszustand herrscht.

9. Therapeutisches Gerät nach einem der Ansprüche 3 bis 8, bei dem das Reaktionsmittel dazu dient, eine Stimulation für den Träger bereitzustellen.

10. Therapeutisches Gerät nach Anspruch 9, bei dem die Stimulation nur durch den Träger feststellbar ist.

11. Therapeutisches Gerät nach Anspruch 10, bei dem die Stimulation von Reaktionsmitteln bereitgestellt wird, die eine Vibration erzeugen können, auf die das Tastgefühl des Trägers reagieren kann.

12. Therapeutisches Gerät nach Anspruch 11, bei dem die Vibration eine Unterhörfrequenz und/oder -lautstärke hat, so daß sie von anderen Personen nicht wahrgenommen werden kann.

13. Therapeutisches Gerät nach einem der Ansprüche 9 bis 12, bei dem weiterhin ein Zählwerk oder dergleichen vorgesehen ist, das ausgebildet ist, um die Zahl der Stimulationen, die auf den Träger von dem Reaktionsmittel ausgeübt werden, aufzuzeichnen.

## Revendications

1. Procédé de prévention et de traitement des déficiences posturales de la colonne vertébrale, du type comprenant le contrôle en continu de la posture d'une personne, la détection du début de prise d'une mauvaise posture, et le signalement de la mauvaise posture détectée, caractérisé par le fait que :
on contrôle de façon continuelle une pluralité de points sur une partie de la colonne vertébrale en détectant, au moyen d'une pluralité de contacts, la relation positionnelle entre lesdits points contrôlés adjacents, et
on signale à l'utilisateur lorsque ladite relation détectée indique une condition de mauvaise posture de ladite partie.

2. Procédé selon la revendication 1, dans lequel ledit signalement est fourni par des moyens de stimulus vibrotactile à fréquence sous-audible.

3. Appareil thérapeutique comprenant:
un organe formant corps (10),
des moyens de détection (14) associés audit organe formant corps (10),
caractérisé en ce que :
ledit organe formant corps (10) est susceptible d'être fixé en un premier point sur une partie choisie de la colonne vertébrale du porteur, lesdits moyens de détection (14) sont susceptibles de contrôler, au moyen d'une pluralité de contacts, au moins un autre point de ladite partie sélectionnée, et
ledit appareil comprend en outre des moyens réactifs (16) répondant aux positions relatives dudit organe formant corps (10) et desdits moyens respectifs de détection (14) et qui sont susceptibles de réagir à une position relative choisie précitée.

4. Appareil thérapeutique selon la revendication 3, dans lequel ledit organe formant corps comprend une bande ou analogue susceptible d'être disposée le long d'une partie de la colonne vertébrale d'un porteur.

5. Appareil thérapeutique selon l'une quelconque des revendications 3 ou 4, dans lequel lesdits moyens de fixation sont choisis parmi des patins adhésifs, des rubans adhésifs, ou des sangles.

6. Appareil thérapeutique selon la revendication 5, dans lequel lesdits moyens de détection comprennent des moyens pour fournir de façon continue un signal ou une autre information de sortie reflétant la courbure de la colonne vertébrale du porteur.

7. Appareil thérapeutique selon la revendication 5, dans lequel lesdits moyens de détection fournissent un signal en réponse à la courbure de la colonne vertébrale du porteur lorsqu'elle excède des limites prédéterminées.

8. Appareil thérapeutique selon la revendication 7, dans lequel lesdits moyens de détection comprennent un jeu de contacts électriques montés à l'intérieur ou sur l'organe formant corps de telle façon que lesdits contacts puissent être actionnés lorsqu'apparaît une condition de posture néfaste.

9. Appareil thérapeutique selon l'une quelconque des revendications 3 à 8, dans lequel lesdits moyens de réaction servent à fournir un stimulus au porteur.

10. Appareil thérapeutique selon la revendication 9, dans lequel ledit stimulus peut être détecté seulement par le porteur.

11. Appareil thérapeutique selon la revendication 10, dans lequel ledit stimulus est fourni par des moyens de réaction susceptibles d'engendrer une vibration à laquelle peut répondre une sensation tactile du porteur.

12. Appareil thérapeutique selon la revendication 11, dans lequel ladite vibration présente un volume et/ou une fréquence sous-audibles de telle façon que la vibration ne puisse être détectée par les autres.

13. Appareil thérapeutique selon l'une quelconque des revendications 9 à 12, dans lequel il est en outre prévu un compteur ou analogue, susceptible d'enregistrer le nombre des stimulations appliquées au porteur par lesdits moyens réactifs.
